# EUROPÄISCHE PATENTANMELDUNG

(11) **EP 4 272 724 A1**
(43) Veröffentlichungstag der Anmeldung: **08.11.2023**
(21) Anmeldenummer: 22171536.0
(22) Anmeldetag: 04.05.2022
(51) Int. Cl.: A61K 8/55, A61K 8/98, A61Q 19/06, A61Q 19/08

(54) **WIRKSTOFFKOMPLEX FÜR KOSMETISCHE ZUBEREITUNGEN**

(71) Anmelder: La Prairie Group AG, 8604 Volketswil (CH)
(72) Erfinder: Dudler, Bernhard, 8340 Hinwil (CH); Stangl, Daniel, 6045 Meggen (CH)
(74) Vertreter: Hartmann, Jost

(57) **Zusammenfassung**

Vorliegende Anmeldung betrifft einen Wirkstoffkomplex und kosmetische Zusammensetzungen.

## Beschreibung

Die vorliegende Erfindung betrifft einen Wirkstoffkomplex für eine kosmetische Zubereitung.

Ein bekanntes Ärgernis für viele Frauen und Männer ist, dass die Haut, insbesondere die Gesichtshaut, mit zunehmendem Alter an Volumen und Festigkeit verliert. Als Folge können sich unter Umständen die Gesichtskonturen sichtbar verändern. Ein Resultat ist die unerwünschte Faltenbildung.

Eine Vielzahl an kosmetischen Wirkstoffen und Zubereitungen sind im Markt, welche bei mehrmaliger, täglicher Anwendung auf der Haut dazu führen, dass sich die Faltentiefe reduziert. Jedoch ist zur wirksamen Behandlung von Falten ein fundamentales Verständnis des Hautaufbaus entscheidend.

Gewebe bestehen hauptsächlich aus zwei unterschiedlichen Elementen: lebenden Zellen und der extrazellulären Matrix. Die extrazelluläre Matrix (ECM) fungiert als eine Art Füllmaterial, das zwischen den ansonsten dicht gepackten Zellen liegt. Die als Bindegewebe bekannte ECM kann auch dazu dienen, Gewebe wie Haut und Muskeln zu verbinden.

Die ECM ist eine komplexe Struktur, die aus verschiedenen Proteinen, Proteoglykanen und Polysacchariden besteht, die ein Gerüst für Zellen bietet, Wasser zurückhält und biologische Prozesse wie Zelladhäsion, Migration, Reparatur, Überleben und Entwicklung moduliert.

Die Rolle der ECM bei der Zelladhäsion und der Signalübertragung in die Zellen wird von Integrinen übernommen, die Signale durch die Zellmembran übertragen, um die intrazelluläre Signalübertragung zu aktivieren. Dieses Übersprechen zwischen der ECM und den Zellen reguliert viele zelluläre Aktivitäten, die wichtig sind, um die Homöostase des Gewebes aufrechtzuerhalten.

In der Haut bildet die ECM eine spezifische Struktur, bestehend aus starken horizontalen und vertikalen Elementen und einer netzartigen Feinstruktur dazwischen, die die räumliche Verteilung der Hautzellen in den verschiedenen Schichten (Epidermis , Dermis und Hypodermis).

Die Retinacula cutis sind Bindegewebsstränge, welche die Subkutis durchziehen und eine Verankerung der Haut an tiefer gelegenen Strukturen (Periost, Faszien) bewirken. Sie schränken die Verschieblichkeit der Haut ein. Die Retinacula cutis bilden ein kontinuierliches Netzwerk aus faseriger extrazellulärer Matrix in der Hypodermis. Die Faserbündel bestehen aus einer Ansammlung von Fasern, die mehr oder weniger vertikal von der äußeren Schicht der tiefen Faszie verlaufen und dünne Verbindungen mit der Basis der Dermis bilden. Nicht alle Fasern erstrecken sich über die volle Dicke der Hypodermis. Horizontale Äste unterteilen die Hypodermis in zwei oder drei Schichten, auch oberflächliche Faszien genannt. Wenn Blutgefäße oder Nerven vorhanden sind, bilden die Fasern der Hautbänder eine faserige Hülle um diese Strukturen.

Zwischen den Fasern dient ein feines Netz aus extrazellulären Matrixelementen der Befestigung der Zellen des Fettgewebes, hauptsächlich Adipozyten und Präadipozyten, an den Hautbändern und wirkt als Gerüst, das die räumliche Verteilung der Zellen strukturiert.

Hautbänder können sehr vereinfacht durch einen Baum visualisiert werden, wobei die Fasern den Stamm und die starken Äste eines Baums darstellen und das feine Netz von ECM-Elementen alle dünnen Äste eines Baums sind.

Die Fasern der Hautbänder werden hauptsächlich von Kollagen I und Elastin gebildet, ergänzt durch Proteoglykane wie Mimecan, Decorin, Prolargin, Lumican und Biglycan.

Die Dichte und Stärke der Hautbänder (Retinacula cutis ) nimmt mit dem Alter ab. Es hat sich gezeigt, dass die Konzentrationen von Schlüsselkomponenten der ECM, die Hautbänder bilden, wie Kollagen I, Kollagen VI und Mimecan, mit zunehmendem Alter abnehmen. Dies wiederum kann zum Auftreten von Alterserscheinungen wie Schlaffheit, Falten und Festigkeits- und wahrscheinlich auch Volumenverlust führen.

Entsprechend sind in der Literatur eine Vielzahl an Versuchen bekannt, die Expression von Kollagen I zu fördern. Ein bekanntes Beispiel ist die US 7279329 B2.

Die bekannten Versuche dürfen jedoch nicht darüber hinwegtäuschen, dass weiterhin ein Bedarf an besonders effektiven Wirkstoffen und Stoffkombination besteht, die die Expression von Kollagen I zu verbessern und so das Auftreten von Hautalterserscheinungen wie Schlaffheit, Falten und Festigkeits- und Volumenverlust reduziert wird.

Überraschend konnte nun gefunden werden, dass die Expression von Kollagen I durch den Gegenstand der vorliegenden Erfindung in synergistischer Weise gesteigert werden konnte.

Ein Gegenstand der vorliegenden Erfindung ist eine Wirkstoffkombination enthaltend
a) mindestens einen Kaviarextrakt, und
b) Mannosephosphat und/oder Natrium Mannosephosphat.

Auch Gegenstand der Erfindung ist eine kosmetische Zubereitung enthaltend die erfindungsgemäße Wirkstoffkombination.

Auch Gegenstand der Erfindung ist die Verwendung der Wirkstoffkombination in kosmetischen Zubereitungen.

Auch Gegenstand der Erfindung ist die Verwendung der Wirkstoffkombination zur Steigerung der Expression von Kollagen I sowie zur Reduktion von Hautalterserscheinungen, insbesondere zur Reduktion von Hautalterserscheinungen, wie Schlaffheit, Falten und Festigkeits- und Volumenverlust.

Ein weiterer Gegenstand der Erfindung ist die Verwendung der Wirkstoffkombination zur Stärkung der Hautbänder bzw. der Retinacula cutis und/oder der Bindegewebsstränge der menschlichen Haut. Stärkung bedeutet in diesem Zusammenhang, dass die Bindegewebsstränge weniger gefestigt werden und so ein Ausleiern der des Gewebes vermindert wird.

Die Hautbänder sind unter dem englischen Begriff "skin ligament" bekannt. Diese werden auch als Retinaculum cutis bezeichnet. Die Retinacula cutis sind Bindegewebsstränge, welche die Subkutis durchziehen und eine Verankerung der Haut (Cutis) an tiefer gelegenen Strukturen (Periost, Faszien) bewirken. Sie schränken die Verschieblichkeit der Haut ein.

Durch die Verstärkte Expression von Kollagen I entsteht eine direkte Stärkung der Skin Ligaments, so dass es zur Reduktion von Hautalterserscheinungen, wie Schlaffheit, Falten und Festigkeits- und Volumenverlust, kommt.

Weiterhin wurde überraschend festgestellt, dass die Wirkstoffkombination der Erfindung ebenfalls die Expression von Thrombospondin-1 intensiv fördert. Thrombospondin-1 ist ein adhäsives Protein, welches Zell-zu-Zell- und Zell-zu-ECM-Wechselwirkungen vermittelt. Im Fettgewebe unterstützt es die Anheftung von Adipozyten an die Hautbänder. Folglich dient es der Anheftung von Bestandteilen der Haut. Eine erhöhte Expression fördert somit den Hautzustand, durch verbesserte Anheftung von Adipozyten an die Hautbänder.

Alle nachfolgend aufgeführten Gewichtsprozentangaben (Gew.-%) beziehen sich, sofern nicht anders angegeben, jeweils auf das Gesamtgewicht der kosmetischen Zubereitung. Wird nachfolgend von Verhältnissen gesprochen, so beziehen diese sich, sofern nicht anders angegeben, auf Gewichtsverhältnisse.

Sofern nicht anders angegeben wurden alle Versuche und Verfahrensschritte unter Normalbedingungen durchgeführt. Der Begriff "Normalbedingungen" bedeutet 20°C, 1013 hPa und eine relative Luftfeuchtigkeit von 50%.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäße Wirkstoffkombination, die erfindungsgemäße Zubereitung sowie auf die erfindungsgemäße Verwendung.

Wird nachfolgend der Begriff Haut verwendet, so bezieht sich dieser ausschließlich auf die menschliche Haut.

Als Glycole oder Glykole werden jene Dialkohole (zweiwertige Alkohole) bezeichnet, die sich vom Ethylenglycol ableiten (sogenannte 1,2-Diole oder vicinale Diole). Beispiele hierfür sind Ethylenglycol und Propylenglycol.

Erfindungsgemäß umfasst die Wirkstoffkombination mindestens einen Kaviarextrakt. Kaviarextrakte werden unter der INCI Bezeichnung "Caviar Extract" in kosmetischen Produkten deklariert.

Vorteilhaft beträgt der Gesamtgehalt an Kaviarextrakt in der erfindungsgemäßen kosmetischen Zubereitung von 0,1 bis 5 Gew.-%, bevorzugt 0,2 bis 4 Gew.-% und insbesondere bevorzugt 0,5 bis 3,5 Gew.-%, bezogen auf das Gesamtgewicht der Zubereitung.

Generell sind Kaviarextrakte unter verschiedenen Handelsnamen kommerziell erhältlich. Ein Beispiel ist Creanatural^{®} Caviar Sevruga Extract hergestellt von The Innovation Company. Weitere Kaviarextrakte können von der mibelle biochemistry group kommerziell bezogen werden.

Erfindungsgemäß sind unter Kaviarextrakt Extrakte von Stör-Fischeiern zu verstehen. Bekannt sind unter anderem der Sibirische Stör, der Kurznasen-Stör, der Jangtse-Stör, See-Stör, der Russische Stör oder auch Waxdick, der Grüne Stör, der Sachalin-Stör, der Adriatische Stör, der Glattdick, der Atlantische Stör, der Persische Stör, der Sterlet, der Amur-Stör, der Chinesische Stör, der Sternhausen, der Europäische Stör, der Weiße Stör, der Kaluga-Hausen und der Europäische Hausen, welcher oftmals als Beluga-Stör bezeichnet wird. Anzumerken ist hier, dass eine Vielzahl an Störarten vom Aussterben bedroht sind, so dass auf den Einsatz von diesen verzichtet werden sollte. Als erfindungsgemäß bevorzugteste werden die Fischeier des Weißen Störs (Acipenser transmontanus) und/oder des Sibirische Störs (Acipenser baerii) gewählt. Der Weiße Stör gilt gemäß der IUCN (International Union for Conservation of Nature and Natural Resources) als nicht gefährdet.

Es ist insbesondere vorteilhaft, wenn ausschließlich Fischeier von Zuchtstören, insbesondere von gezüchteten Sibirischen und/oder Weißen Stören zur Herstellung des Kaviarextrakts eingesetzt werden. Es ist weiterhin äußerst bevorzugt, wenn die Fischeier in einem für den Fisch nicht letalen Verfahren gewonnen werden.

Generell erfolgt die Herstellung von erfindungsgemäßen Kaviarextrakten vorteilhaft nach einem Verfahren umfassend die Schritte
1) Homogenisierung der bereitgestellten Fischeier in mindestens einem Lösungsmittel,
2) Extraktion mindestens einer flüssigen Phase aus dem unter 1. erhaltenen Homogenisat, und
3) Ggf. einer Filtration des unter 2) gewonnenen Extrakts, um feste Schwebstoffe aus dem Extrakt zu entfernen.

Weitere Aufreinigungsschritte sind generell möglich.

Es ist erfindungsgemäß vorteilhaft, wenn mindestens ein Kaviarextrakt in der Wirkstoffkombination enthalten ist, bei dessen Herstellung mindestens Wasser und/oder ein Glykol als Lösemittel bei der Homogenisation der Fischeier zugesetzt werden und bei dem die wässrige Phase zum Erhalt des Kaviarextrakts extrahiert wird.

Es ist erfindungsgemäß vorteilhaft, wenn mindestens ein Kaviarextrakt eingesetzt wird, der dadurch gekennzeichnet, dass bei dessen Herstellung mindestens Wasser jedoch kein Glykol oder Glykol in Gewichtsanteilen von Wasser zu Glykol von 1,1:1,0 bis 1000:1,0 als Lösemittel bei der Homogenisation der Fischeier zugesetzt werden und bei dem die wässrige Phase zum Erhalt des Kaviarextrakts extrahiert wird. Dieser Extrakt wird als hydrophiler Kaviarextrakt bezeichnet.

Es ist erfindungsgemäß vorteilhaft, wenn mindestens ein Kaviarextrakt dadurch gekennzeichnet ist, dass bei dessen Herstellung mindestens Glykol und optional Wasser als Lösemittel bei der Homogenisation der Fischeier als Lösemittel zugesetzt werden, wobei der Gewichtsanteilen von Wasser zu Glykol von 1,0:1,0 bis 1,0:1000 beträgt, und bei dem die glykolische Phase zum Erhalt des Kaviarextrakts extrahiert wird. Die glykolische Phase ist die Phase, die Glykol enthält. Dieser Extrakt wird als glykolischer Kaviarextrakt bezeichnet.

Weiterhin ist es erfindungsgemäß vorteilhaft, wenn mindestens ein Kaviarextrakt in der Wirkstoffkombination enthalten ist, bei dessen Herstellung mindestens Öl als Lösemittel bei der Homogenisation der Fischeier zugesetzt werden und bei dem die Ölphase extrahiert wird. Entsprechend wird eine Ölphase erhalten, die den Kaviarextrakt enthält. Vorteilhaft werden dabei als Öle Triglyceride eingesetzt, wobei Caprylic/Capric Triglyceride insbesondere bevorzugt ist.

Es ist insbesondere vorteilhaft mindestens zwei verschiedene Kaviarextrakte einzusetzen, wobei ein erster lipophiler Kaviarextrakt eingesetzt wird, bei dessen Herstellung mindestens Öl, vorteilhaft Caprylic/Capric Triglyceride, als Lösemittel bei der Homogenisation der Fischeier zugesetzt werden und bei dem die Ölphase zum Erhalt des Extrakt extrahiert wird, und wobei ein zweiter hydrophiler oder glykolischer Kaviarextrakt eingesetzt wird, bei dessen Herstellung mindestens Wasser und/oder ein Glykol als Lösemittel bei der Homogenisation der Fischeier zugesetzt werden und bei dem die wässrige Phase zum Erhalt des zweiten Extrakts extrahiert wird.

Es ist weiterhin insbesondere vorteilhaft, wenn drei verschiedene Kaviarextrakte eingesetzt werden, wobei
- ein erster lipophiler Kaviarextrakt eingesetzt wird, bei dessen Herstellung mindestens Öl, vorteilhaft Caprylic/Capric Triglyceride, als Lösemittel bei der Homogenisation der Fischeier zugesetzt werden und bei dem die Ölphase zum Erhalt des lipophilen Kaviarextrakts extrahiert wird,
- ein zweiter wässriger Kaviarextrakt eingesetzt wird, bei dessen Herstellung mindestens Wasser, jedoch kein Glykol, als Lösemittel bei der Homogenisation der Fischeier zugesetzt werden und bei dem die wässrige Phase zum Erhalt des wässrigen Kaviarextrakts extrahiert wird, und
- ein dritter glykolischer Kaviarextrakt eingesetzt wird, bei dessen Herstellung mindestens Glykol als Lösemittel bei der Homogenisation der Fischeier zugesetzt werden und bei dem die glykolische Phase zum Erhalt des dritten Kaviarextrakts extrahiert wird.

Eine glykolische Phase ist eine Phase aufweisend Glykol.

Es ist weiterhin insbesondere vorteilhaft, wenn drei verschiedene Kaviarextrakte eingesetzt werden, wobei
- ein erster lipophiler Kaviarextrakt, wie oben definier,
- ein zweiter wässriger Kaviarextrakt, wie oben definiert, und
- ein dritter glykolischer Kaviarextrakt, wie oben definiert
gewählt werden. Durch die Kombination konnten überraschend vorteilhafte Ergebnisse in Bezug auf die Erfindung erzielt werden.

Bezüglich des lipophilen Kaviarextrakts ist es bevorzugt, wenn neben dem Öl, welches vorteilhaft Caprylic/Capric Triglyceride ist, Wasser bei der Homogenisations zugesetzt wird. In diesem Fall ergibt sich eine Trennung der lipophilen und hydrophilen Bestandteile des Homogenisats. Entsprechend werden bei der Extraktion der Ölphase vorwiegend lipophile Bestandteile des Homogenisats extrahiert. In diesem Verfahren ist es vorteilhaft, wenn bei den zugesetzten Ölen zur Homogenisation, mindestens Caprylic/Capric Triglyceride enthalten ist und weiterhin vorteilhaft der Anteil von Caprylic/Capric Triglyceride mindestens 90 Gew.-% bezogen auf das Gesamtgewicht aller zugesetzten Öle beträgt. Weiterhin ist es vorteilhaft, wenn der Gewichtsanteil der Fischeier zur zugegebenen Ölphase bei der Homogenisation von 1:0,1 bis 1:1 und insbesondere von 1:0,2 bis 1:0,5 beträgt. Sofern Wasser für die Homogenisation zugesetzt wird, beträgt der Gewichtsanteil der Fischeier zu der durch Zugabe von Wasser entstandenen wässrigen Phase von 1:10 bis 1:1, bevorzugt von 1:8 bis 1:2 und insbesondere bevorzugt von 1:5 bis 1:3.

Bezüglich des wässrigen Kaviarextrakts ist es bevorzugt, wenn neben dem Wasser zusätzlich mindestens ein Öl, vorteilhaft Caprylic/Capric Triglyceride, bei der Homogenisations zugesetzt wird. In diesem Fall ergibt sich eine Trennung der lipophilen und hydrophilen Bestandteile des Homogenisats. Entsprechend werden bei der Extraktion der Wasserphase vorwiegend hydrophile Bestandteile des Homogenisats extrahiert. In diesem Verfahren ist es vorteilhaft, wenn bei dem zugesetzten Öl zur Homogenisation, mindestens Caprylic/Capric Triglyceride enthalten ist und weiterhin vorteilhaft der Anteil von Caprylic/Capric Triglyceride mindestens 90 Gew.-% bezogen auf das Gesamtgewicht aller zugesetzten Öle beträgt. Weiterhin ist es vorteilhaft, wenn der Gewichtsanteil der Fischeier zur zugegebenen Ölphase bei der Homogenisation von 1:0,1 bis 1:1 und insbesondere von 1:0,2 bis 1:0,5 beträgt. Vorteilhaft beträgt der Anteil der Fischeier zu der wässrigen Phase von 1:10 bis 1:1, bevorzugt von 1:8 bis 1:2 und insbesondere bevorzugt von 1:5 bis 1:3. Per se ist es vorteilhaft, wenn der wässrige Kaviarextrakt frei von Glycolen ist, oder die Anteile von Glykol weniger als 10 Gew.-% bezogen auf das Gesamtgewicht der Extrakts ausmacht. Vorteilhaft wird kein Glykol bei bzw. zur Homogenisation und Extraktion zugesetzt.

Bezüglich des glycolischen Kaviarextrakts ist es bevorzugt, wenn Propylenglycol bei der Homogenisations zugesetzt wird. Neben Propylenglykol ist es weiterhin vorteilhaft, wenn ebenfalls Wasser zugesetzt wird. Vorteilhaft beträgt der Anteil von Propylenglykol zu Wasser von 20:1 bis 1:1, bevorzugt 12:1 bis 2:1 und insbesondere bevorzugt 10:1 bis 5:1. In diesem Fall werden Bestandteile des Homogenisats in der glycolischen Lösung gelöst, welchen ggf. in reinem Wasser nicht löslich sind. Nach der Homogensiation wird die glycolische Phase extrahier. Weiterhin ist es vorteilhaft, wenn der Gewichtsanteil der Fischeier zum zugegebenen Glycol, insbesondere zu Propylenglycol von 1:0,1 bis 1:100 und insbesondere von 1:0,2 bis 1:20 und insbesondere 1:0,5 bis 1:10 beträgt.

Die Wirkstoffzusammensetzung variiert ggf. abhängig vom beschriebenen Extraktionsverfahren.

Vorteilhaft beträgt in der obigen Ausführung das Gewichtsverhältnis vom lipophilen Kaviarextrakt zum wässriger Kaviarextrakt von 50:1 bis 1:10, bevorzugt 30:1 bis 1:1 und insbesondere bevorzugt 15:1 bis 5:1.

Vorteilhaft beträgt in der obigen Ausführung das Gewichtsverhältnis vom lipophilen Kaviarextrakt zum glykolischen Kaviarextrakt von 50:1 bis 1:50, bevorzugt 20:1 bis 1:20 und insbesondere bevorzugt 10:1 bis 1:10.

Neben Kaviarextrakt umfasst die erfindungsgemäße Wirkstoffkombination mindestens Mannosephosphat und/oder Natrium Mannosephosphat.

Es ist insbesondere bevorzugt, wenn als Natrium Mannosephosphat das Natrium Mannose-6-Phosphat enthalten ist. Dieses ist kommerziell unter der Bezeichnung Sodium mannose 6-phosphate von Sigma Aldrich erhältlich.

Es ist ebenfalls bevorzugt, wenn als Mannosephosphat Mannose-6-phosphat enthalten ist.

Vorteilhaft beträgt das Gewichtsverhältnis von der Gesamtheit der erfindungsgemäßen Kaviarextrakt zu Mannosephosphat und/oder Natrium Mannosephosphat von 200:1 bis 1:50, bevorzugt 100:1 bis 1:10, bevorzugt von 50:1 bis 1:1 und insbesondere bevorzugt von 30:1 bis 2:1.

Vorteilhaft beträgt das Gewichtsverhältnis von der Gesamtheit der erfindungsgemäßen Kaviarextrakt zu Mannose-6-phosphat und/oder Natrium Mannose-6-phosphat von 200:1 bis 1:50, bevorzugt 100:1 bis 1:10, bevorzugt von 50:1 bis 1:1 und insbesondere bevorzugt von 30:1 bis 2:1.

Zusätzlich ist es erfindungsgemäß vorteilhaft, wenn die Wirkstoffkombination Mannose umfasst. Vorteilhaft beträgt das Gewichtsverhältnis von der Gesamtheit der erfindungsgemäßen Kaviarextrakte zu Mannose von 200:1 bis 1:50, bevorzugt 100:1 bis 1:10, bevorzugt von 50:1 bis 1:1 und insbesondere bevorzugt von 30:1 bis 2:1.

Weiterhin ist es vorteilhaft, wenn Natrium Mannosephosphat und/oder Mannosephosphat zusammen mit 1,2,3-Propantriol eingesetzt wird. Entsprechend ist 1,2,3-Propantriol vorteilhaft enthalten.

Die erfindungsgemäße Wirkstoffkombination wird vorteilhaft in den verschiedensten kosmetischen Zubereitungen eingesetzt. Diese sind ebenfalls erfindungsgemäß.

Die erfindungsgemäßen kosmetischen Zubereitungen können in den üblichen kosmetischen galenischen Zubereitungsformen, bevorzugt als Gel, O/W-Emulsion, W/O-Emulsion, W/O/W-Emulsion, O/W/O-Emulsion, Mikroemulsion, kosmetischer Stick vorliegen.

Die erfindungsgemäßen kosmetischen Zubereitungen können, bevorzugt als Emulsion, Salbe, Foundation, Toner, wässrige Lösung, Creme, Gel, Puder, Maske, Schaumzubereitung und Aerosolzubereitung vorliegen.

Die erfindungsgemäße Wirkstoffkombination ist in kosmetischen Zubereitungen vorteilhaft in Anteilen von 0,01 bis 5 Gew.-%, bevorzugt von 0,02 bis 4 Gew.-% und insbesondere bevorzugt von 0,05 bis 3 Gew.-%, bezogen auf das Gesamtgewicht der kosmetischen Zubereitung, enthalten.

Kosmetische Zubereitungen, die zur täglichen Pflege auf die Gesichtshaut aufgetragen werden, sind zumeist als Emulsionen formuliert. Unter Emulsionen versteht man im Allgemeinen heterogene Systeme, die aus zwei nicht oder nur begrenzt mischbaren Flüssigkeiten bestehen, wobei eine der beiden Flüssigkeiten in Form feinster Tröpfchen in der anderen Flüssigkeit dispergiert ist. Mit dem bloßen Auge erscheint eine Emulsion als homogen. Sind beide Flüssigkeiten Wasser und Öl, und liegt das Öl als fein verteilte Tröpfchen im Wasser vor, so handelt es sich um eine Öl-in-Wasser-Emulsion (O/W-Emulsion). Liegt hingegen das Wasser als fein verteilte Tröpfchen im Öl vor, so handelt es sich um eine Wasserin-ÖI-Emulsion (W/O-Emulsion).

Erfindungsgemäß besonders vorteilhaft, wenn die kosmetische Zubereitung in Form einer O/W-Emulsion vorliegt.

Emulgatoren dienen dazu, Emulsionen zu stabilisieren. Stabilisieren heißt in diesem Zusammenhang, dass die Phasenseparation der Emulsion verhindert bzw. verzögert wird.

Dementsprechend lassen sich durch Einsatz von entsprechend ausgewählten Emulgatorsystemen stabile Emulsionen herstellen.

Emulgatoren sind Moleküle mit einem polaren, hydrophilen Strukturelement und einem unpolaren, lipophilen Strukturelement. Allgemein können derartige Moleküle über den HLB-Wert (dimensionslose Zahl zwischen 0 und 20) definiert werden, der angibt, ob eine bevorzugte Wasser- oder Öllöslichkeit vorliegt. Wasser in Öl Emulgatoren (W/O-Emulgatoren) weisen üblicher Weise einen HLB-Wert im Bereich von 3 bis 8 auf. Dementsprechend fördern W/O-Emulgatoren die Stabilisierung von einer wässrigen Phase, die suspendiert in einer Ölphase vorliegt. Öl-in-Wasser Emulgatoren (O/W-Emulgatoren) weisen einen HLB-Wert größer 8 bis 18 auf. Diese fördern die Stabilisierung einer Ölphase, die suspendiert in einer wässrigen Phase vorliegt.

Liegt die kosmetische Zubereitung als Öl-in-Wasser Emulsion vor, so ist es vorteilhaft, wenn die kosmetische Zubereitung mindestens einen O/W-Emulgator mit einem HLB-Wert im Bereich von größer 8 bis 18 enthält. Vorteilhaft zu wählende O/W-Emulgatoren sind beispielsweise der folgenden Auflistung zu entnehmen:
HLB-Wert Chemische Bezeichnung
- 8,2: Triglycerinmonooleat
- 8,3: Diethylenglycolmonolaurat
- 8,4: Polyoxyethylen(4)cetylether Polyoxyethylenglycol(400)dioleat
- 8,5: Natriumcaproyllactylat Polyethylenglycol(200)monostearat Sorbitanmonooleat
- 8,6: Sorbitanmonolaurat Polyethylenglycol(200)monolaurat
- 8,8: Polyoxyethylen(4)myristylether Polyethylenglycol (400)dioleat
- 8,9: Nonylphenol, polyoxyethyliert mit 4 Mol EO
- 9,0: Oleth-5
- 9,2 - 9,7: Polyoxyethylen(4)laurylalkohol
- 9,3: Polyoxyethylen(4)tridecylalkohol
- 9,6: Polyoxyethylen(4)sorbitanmonostearat
- 9,8: Polyethylenglycol(200)monolaurat
- 10-11: Polyethylenglycol(400)monooleat
- 10,0: Didodecyldimethylammoniumchlorid
- 10,0: Polyethylenglycol(200)monolaurat Polyethylenglycol(400)dilaurat Polyethylenglycol(600)dioleat Polyoxyethylen(4)sorbitanmonostearat Polyoxyethylen(5)sorbitanmonooleat
- 10-12: Glyceryl Stearate Citrate
- 10,2: Polyoxyethylen(40)sorbitol hexaoleat
- 10,4 - 10,6: Polyoxyethylenglycol(600)distearat
- 10,5: Polyoxyethylen(20)sorbitantristearat
- 10,6: Saccharosemonostearat
- 10,7: Saccharosemonooleat

- 11 - 11,4: Polyethylenglycol(400)monooleat
- 11,0: Polyethylenglycol(350)monostearat Polyethylenglycol(400)monotallat Polyoxyethylenglycol(7)monostearat Polyoxyethylenglycol(8)monooleat Polyoxyethylen(20)sorbitantrioleat Polyoxyethylen(6)tridecylalkohol
- 11,1: Polyethylenglycol(400)monostearat
- 11,2: Polyoxyethylen(9)monostearat Saccharosemonooleat Saccharosemonostearat
- 11,4: Polyoxyethylen(50)sorbitol hexaoleat Saccharosemonotallat Saccharosestearatpalmitat
- 11,6: Polyoxyethylenglycol(400)monoricinoleat
- 11,7: Saccharosemonomyristat Saccharosemonopalmitat
- 12,0: PEG-10 Soy Sterol Triethanolaminoleat
- 12,2-12,3: Nonylphenol, ethoxyliert mit 8 Mol EO
- 12,2: Saccharosemonomyristat
- 12,4: Saccharosemonolaurat Polyoxyethylen(10)oleylalkohol, Polyoxyethylen(10)oleylether Polyoxyethylen(10)stearylalkohol, Polyoxyethylen(10)stearylether
- 12,5: Polyoxyethylen(10)stearylcetylether
- 12,7: Polyoxyethylen(8)tridecylalkohol
- 12,8: Polyoxyethylenglycol(400)monolaurat Saccharosemonococoat
- 12,9: Polyoxyethylen(10)cetylether
- 13: Glycerinmonostearat, ethoxyliert (20 Mol EO)
- 13,0: Eumulgin O 10 (Polyoxyethylen(10)oleylether) Eumulgin 286 (Nonoxynol-10) Eumulgin B 1 (Ceteareth-12)
- 13,0: C12-Fettamine, ethoxyliert (5 Mol EO)
- 13,1: Nonylphenol, ethoxyliert (9,5 Mol EO)
- 13,2: Polyethylenglycol(600)monostearat Polyoxyethylen(16)tallöl
- 13,3: Polyoxyethylen(4)sorbitanmonolaurat
- 13,5: Nonylphenol, ethoxyliert (10,5 Mol EO) Polyethylenglycol(600)monooleat
- 13,7: Polyoxyethylen(10)tridecylalkohol Polyethylenglycol(660)monotallat Polyethylenglycol(1500)monostearat Polyoxyethylenglycol(1500)dioleat
- 13,9: Polyethylenglycol(400)monococoat Polyoxyethylen(9)monolaurat
- 14-16: Ricinusöl, mit 40 EO ethoxyliert und hydriert
- 14,0: Polyoxyethylen(12)laurylether Polyoxyethylen(12)tridecylalkohol
- 14,2: Polyoxyethylen(15)stearylalkohol
- 14,3: Polyoxyethylen(15)stearylcetylether
- 14,4: Gemisch aus C12-C15-Fettalkoholen mit 12 Mol EO
- 14,5: Polyoxyethylen(12)laurylalkohol
- 14,8: Polyoxyethylenglycol(600)monolaurat
- 14,9 - 15,2: Sorbitanmonostearat, mit 20 EO ethoxyliert
- 15 - 15,9: Sorbitanmonooleat, mit 20 EO ethoxyliert
- 15,0: PEG-20 Glyceryl Stearate PEG-40 Castor Oil Decylglucosid Dodecylglucosid Dodecyltrimethylammoniumchlorid Nonylphenol, ethoxyliert mit 15 Mol EO Polyethylenglycol(1000)monostearat Polyoxyethylen(600)monooleat
- 15-17: Ricinusöl, mit 60 EO ethoxyliert und hydriert
- 15,3: C12-Fettamine, ethoxyliert (5 Mol EO) Polyoxyethylen(20)oleylalkohol, Polyoxyethylen(20)oleylether
- 15,4: Polyoxyethylen(20)stearylcetylether
- 15,5: Polyoxyethylen(20)stearylalkohol
- 15,6: Polyoxyethylenglycol(1000)monostearat Polyoxyethylen(20)sorbitanmonopalmitat
- 15,7: Polyoxyethylen(20)cetylether
- 15,9: Dinatriumtriethanolamindistearylheptaglycolethersulfosuccinat
- 16,0: Nonylphenol ethoxyliert mit 20 Mol EO Polyoxyethylen(25)propylenglycolstearat
- 16 - 16,8: Polyoxyethylen(30)monostearat
- 16,3-16,9: Polyoxyethylen(40)monostearat
- 16,5 - 16,7: Polyoxyethylen(20)sorbitanmonolaurat
- 16,6: Polyoxyethylen(20)sorbit
- 16,7: C18-Fettamine, polyoxyethyliert mit 5 Mol EO Polyoxyethylen(23)laurylalkohol
- 17,0: Ceteareth-30, z. B. Eumulgin B 3 Octylglucosid (Triton CG 110) Polyoxyethylen(30)glycerylmonolaurat
- 17,1: Nonylphenol, ethoxyliert mit 30 Mol EO
- 17,4: Polyoxyethylen(40)stearylalkohol
- 18,8: PEG-100 Stearate Steareth-100
- 19,1: PEG-80 Sorbitan Laurate

In der vorstehenden Auflistung steht die Abkürzung EO für Ethylenoxid und PEG für Polyethylenglykol.

Eine derartige O/W-Emulsion kann erfindungsgemäß vorteilhaft ebenfalls W/O-Emulgatoren enthalten, wobei das Verhältnis der O/W-Emulgatoren zu den W/O-Emulgatoren unter Berücksichtigung der jeweiligen HLB-Werte so zu wählen ist, dass sich eine O/W-Emulsion einstellt. Eine bekannte Mischung an O/W-Emulgatoren und W/O-Emulgatoren ist das Handelsprodukt Arlacel 170 von Croda enthaltend Glyceryl Stearate und PEG-100 Stearate, wobei das Verhältnis der beiden Substanzen so gewählt ist, dass sich ein Gesamt-HLB von etwa 11 ergibt.

Die erfindungsgemäße kosmetische Zubereitung enthält zusätzlich vorteilhaft Öle gewählt aus der Gruppe der Lecithine und der Fettsäuretriglyceride, namentlich der Triglycerinester gesättigter und/oder ungesättigter, verzweigter und/oder unverzweigter Alkancarbonsäuren mit einer Kettenlänge von 8 bis 24, insbesondere 12 bis 18 C-Atomen enthalten. Die Fettsäuretriglyceride können vorteilhaft gewählt werden aus der Gruppe der synthetischen, halbsynthetischen und natürlichen Öle, wie z.B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Des Weiteren kann die erfindungsgemäße kosmetische Zubereitung vorteilhaft Öle enthalten, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Ferner kann die erfindungsgemäße kosmetische Zubereitung vorteilhaft Fett- und/oder Wachskomponenten aus der Gruppe der pflanzlichen Wachse, tierischen Wachse, Mineralwachse und petrochemischen Wachse enthalten. Erfindungsgemäß günstig sind Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), und Syncrowax AW 1 C (C18-36 -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C30-50 -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C20-40-Alkylstearat, C20-40-Alkylhydroxystearoylstearat und/oder Glykolmontanat.

Es kann ebenfalls vorteilhaft im Sinne der vorliegenden Erfindung sein, wenn die kosmetische Zubereitung cyclische, verzweigte und/oder lineare Silicone enthält. Die Gruppe der cyclischen, verzweigten und/oder linearen Silicone werden im Rahmen der vorliegenden Offenbarung auch als "Siliconöle" bezeichnet. Lineare Siliconöle werden mit der INCI Bezeichnung Dimethicone beschrieben und weisen eine Struktur gemäß der Formel (I) 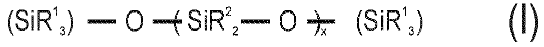 auf, während verzweigte Siliconöle gemäß der Formel (II) 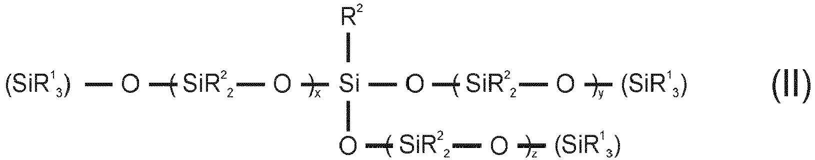 beschrieben werden können, wobei R¹ und R² unabhängig voneinander ein Wasserstoffatom, eine Methylgruppe oder eine lineare oder verzweigte, gesättigte oder ungesättigte Kohlenwasserstoffgruppe mit 3 bis 30 Kohlenstoffatomen sein können, und wobei x, y, und z unabhängig voneinander ganzzahlige Zahlen im Bereich von 0 bis 60000 sind. Cyclische Silicone sind unter der INCI-Bezeichnung Cyclomethicone bekannt.

Vorteilhaft ist es dabei, wenn der Gewichtsanteil der Silikonöle in der kosmetischen Zubereitung 3 Gew.-% bis 10 Gew.-%, bezogen auf das Gesamtgewicht der kosmetischen Zubereitung, beträgt.

Ferner ist die kosmetische Zubereitung vorteilhaft dadurch gekennzeichnet, dass der Gesamtanteil der Ölphase in der O/W-Emulsion 2 bis 30 Gew.-%, bevorzugt 5 Gew.-% bis 25 Gew.-% und insbesondere bevorzugt 8 Gew.-% bis 22 Gew.-%, bezogen auf das Gesamtgewicht der kosmetischen Zubereitung beträgt. Die Silikonöle zählen ebenfalls zur Ölphase der kosmetischen Zubereitung. Emulgatoren zählen definitionsgemäß nicht zur Ölphase der erfindungsgemäßen kosmetischen Zubereitung.

Darüber hinaus ist es vorteilhaft, wenn der Gesamtanteil an Wasser in der erfindungsgemäßen kosmetischen Zubereitung 50 Gew.-% bis 95 Gew.-%, bevorzugt 60 Gew.-% bis 80 Gew.-%, bezogen auf das Gesamtgewicht der kosmetischen Zubereitung, beträgt.

Ferner ist es vorteilhaft, wenn die kosmetische Zubereitung ein oder mehrere Rheologiemodifizierer enthält. Bevorzugt zu wählende Rheologiemodifizierer sind gewählt aus der Gruppe der folgenden INCI Substanzen:
- Carbomer (Carbopole der Typen 980, 981, 2984, 5984 von der Fa. Lubrizol); Acrylates Copolymer (z.B. Carbopol^{®} Aqua SF-1 Polymer von der Fa. Lubrizol), Acrylates/C 10-30 Alkyl Acrylate Crosspolymer (z. B. Pemulen TR 1 , Pemulen TR 2, Carbopol 1328 von der Fa. Lubrizol), Hydroxyethyl Acrylates/Sodium Acryloyldimethyl Taurate Copolymer, Ammonium Acryloyldimethyltaurate/VP Copolymer (z.B. Aristoflex AVC von der Fa. Clariant), Polyacrylate-1 Crosspolymer (z.B. Carbopol^{®} Aqua CC Polymer von der Fa. Lubrizol); Sodium Polyacrylate (z.B. Cosmedia SP von der Fa. BASF); Copolymer aus Vinylpyrrolidon und Acrylsäure
- Cellulosen und Cellulosederivate, z.B. Hydroxypropylmethylcellulose, Methylcellulose, Carboxymethylcellulose, Hydroxyethylcellulose, Hyaluronsäure und Xanthangummi
- Stärken, zum Beispiel Tapiokastärke.

Insbesondere bevorzugt werden die Rheologiemodifizierer gewählt aus der Gruppe der unter der INCI-Bezeichnung bekannten Substanzen Carbomer, Acrylates/C 10-30 Alkyl Acrylate Crosspolymer, Sodium Polyacrylate, Hydroxyethyl Acrylates/Sodium Acryloyldimethyl Taurate Copolymer und Ammonium Acryloyldimethyltaurate/VP Copolymer.

Vorteilhaft beträgt der Gesamtanteil dieser Rheologiemodifizierer, insbesondere der Gesamtanteil der vorstehend als bevorzugt gekennzeichneten Rheologiemodifizierer, 0,05 bis 5 Gew.-%, bevorzugt von 0,1 bis 2,5 Gew.-%, bezogen auf das Gesamtgewicht der kosmetischen Zubereitung. Darüber hinaus ist es insbesondere vorteilhaft, wenn zusätzlich zu den vorstehend als insbesondere bevorzugt genannten Substanzen Tapiokastärke in einem Anteil von bis zu 3,5 Gew.-%, bezogen auf das Gesamtgewicht der kosmetischen Zubereitung, enthalten ist.

Des Weiteren ist es vorteilhaft, wenn das Gewichtsverhältnis aller enthaltenen erfindungsgemäßen Rheologiemodifizierer zu der enthaltenen Ölphase 1:1 bis 1:30, bevorzugt 1:2 bis 1:28, insbesondere bevorzugt 1:20 bis 1:27 beträgt. Derartige erfindungsgemäße kosmetische Zubereitungen weisen eine überraschend vorteilhafte Cremigkeit auf und werden nicht als bröselig oder zu ölig und zu flüssig vom Verbraucher wahrgenommen.

Es ist erfindungsgemäß vorteilhaft, wenn die erfindungsgemäße kosmetische Zubereitung Cetylalkohol, Stearylalkohol oder eine Mischung aus Cetylalkohol und Stearylalkohol enthält.

Enthält die kosmetische Zubereitung Cetylalkohol, Stearylalkohol oder eine Mischung aus Cetylalkohol und Stearylalkohol, so ist es erfindungsgemäß vorteilhaft, wenn der Gesamtanteil dieser Substanzen von 0,5 bis 5,5 Gew.-%, bezogen auf das Gesamtgewicht der kosmetischen Zubereitung, beträgt.

Darüber hinaus ist es vorteilhaft, wenn die erfindungsgemäße kosmetische Zubereitung zusätzlich ein oder mehrere Substanzen gewählt aus der Gruppe Ethanol, Isopropanol, Propylenglykol, Propanediol, Glycerin, Ethylenglykol, Ethylenglykolmonoethyl- oder - monobutylether, Propylenglykolmonomethyl, -monoethyl- oder -monobutylether und/oder Diethylenglykol monomethyl- oder -monoethylether enthält. Dabei ist es bevorzugt, wenn die kosmetische oder dermatologische Zubereitung Glycerin und/oder Propanediol enthält.

Ebenso vorteilhaft ist es die erfindungsgemäßen kosmetischen Zubereitungen als Sonnenschutzmittel zu verwenden. Dementsprechend enthalten die Zubereitungen im Sinne der vorliegenden Erfindung vorzugsweise mindestens eine UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die Zubereitungen gemäß der vorliegenden Erfindung können mindestens eine bei Raumtemperatur flüssige UV-Filtersubstanz enthalten.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol^{®} SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |
| Tioveil AQ 10PG | Alumina / Silica | Solaveil / Uniquema |
| Eusolex T-aqua | Wasser/Alumina/Natriummetaphosphat | Merck |

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex^{®} 9020 verkauft wird.

Vorteilhafte UV-Filtersubstanzen im Sinne der vorliegenden Erfindung sind ferner:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Symrise erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Symrise erhältlich ist;
- 1,4-di(2-oxo-10-Sulfo-3-bornylidenmethyl)-Benzol (auch: 3,3'-(1,4-Phenylendimethy-lene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenme-thyl)benzolsulfonsäure, 2-Methyl-5-(2-oxo-3-bornylidenmethyl)sulfonsäure und deren Salze.
- Benzoxazol-Derivate, wie z. B. das 2,4-bis-[5-1(dimethylpropyl)benzoxazol-2-yl-(4-phe-nyl)-imino]-6-(2-ethylhexyl)-imino-1,3,5-triazin mit der CAS Nr. 288254-16-0, welches bei 3V Sigma unter der Handelsbezeichnung Uvasorb^{®} K2A erhältlich ist.
- Hydroxybenzophenone, z. B. der 2-(4'-Diethylamino-2'-hydoxybenzoyl)-benzoesäure-hexylester (auch: Aminobenzophenon), welcher unter der Handelsbezeichnung Uvinul A Plus bei der Fa. BASF erhältlich ist.
- Triazinderivate, wie z. B. 2,4-Bis-{[4-(2-Ethyl-hexyloxy)-2-hydroxy]-phenyl}-6-(4-methoxy-phenyl)-1,3,5-triazin (INCI: Bis-Ethylhexyloxylphenol Methoxyphenyl Triazin), welches unter der Handelsbezeichnung Tinosorb^{®} S bei der CIBA-Chemikalien GmbH erhältlich ist; Dioctylbutylamidotriazon (INCI: Diethylhexyl Butamido Triazone), welches unter der Handelsbezeichnung UVASORB HEB bei Sigma 3V erhältlich ist; 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL^{®} T 150 vertrieben wird; 2-[4,6-Bis(2,4-dimethylphenyl)-1,3,5-triazin-2-yl]-5-(octyloxy)phenol (CAS Nr.: 2725-22-6).
- Benzotriazole, wie z. B. 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z. B. unter der Handelsbezeichnung Tinosorb^{®} M bei der CIBA-Chemikalien GmbH erhältlich ist.
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter
- Ethylhexyl-2-cyano-3,3-diphenylacrylat (Octocrylen), welches von BASF unter der Bezeichnung Uvinul^{®} N 539 T erhältlich ist.

Besonders vorteilhafte kosmetische Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetra-sulfonsäure-bis-natriumsalz jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Die Gesamtmenge der Filtersubstanzen wird aus dem Bereich von 0,1 bis 30 Gew.-%, vorzugsweise 0,5 bis 10 Gew.-%, insbesondere 1,0 bis 8,0 Gew.-% - jeweils bezogen auf das Gesamtgewicht der kosmetischen Zubereitungen - gewählt, um kosmetische oder dermatologische Zubereitungen zur Verfügung zu stellen, die das Haar bzw. die Haut vor dem gesamten Bereich der ultravioletten Strahlung schützen.

Ferner ist es vorteilhaft, wenn die erfindungsgemäße kosmetische Zubereitung mindestens einen weiteren Wirkstoff zur kosmetischen Behandlung und/oder kosmetischen Prophylaxe unerwünschter Hautpigmentierung enthält.

Die kosmetischen Zubereitungen können dementsprechend ferner weitere kosmetische Hilfsstoffe enthalten, wie sie üblicherweise in solchen Zubereitungen verwendet werden, beispielsweise weitere Konsistenzgeber, Filmbildner, Stabilisatoren, Füllstoffe, Konservierungsmittel, Parfüme, Substanzen zum Verhindern des Schäumens, Farbstoffe, weitere Pigmente, die färbende Wirkung haben, oberflächenaktive Substanzen, weichmachende, anfeuchtende und/oder feuchthaltende Substanzen, entzündungshemmende Substanzen, zusätzliche Wirkstoffe wie Vitamine oder Proteine, Insektenrepellentien, Bakterizide, Viruzide, Salze, antimikrobiell, proteolytisch oder keratolytisch wirksame Substanzen oder andere übliche Bestandteile einer kosmetischen Formulierung wie weitere Alkohole, Polyole, Schaumstabilisatoren, organische Lösungsmittel oder auch Elektrolyte.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Die Wirksamkeit der Erfindung wurde in einem ELISA-Test nachgewiesen. ELISA (enzymelinked immunosorbent assay) ist ein verbreitetes Verfahren, um einzelne Proteine nachweisen zu können. Dabei nutzt man die Mechanismen des Immunsystems: Wird eine Substanz vom Immunsystem als fremd erkannt, bildet es "Antikörper", die an das fremde Molekül andocken und es so markieren.

Zur Durchführung der Experimente wurde Hypodermis (von Mammoplastikoperationen) von einem gesunden Spender (56 Jahre alt) verwendet. Die Hypodermis wurde präpariert und auf ungefähr 200-mg-Stücke gewogen. Die Gewebe wurden im submersen Zustand in definiertem und serumfreiem Hypodermis-Gewebekulturmedium (CB-EM-HYP) in einem aktiv befeuchteten biomedizinischen Inkubator (Memmert, Deutschland) bei 37°C und 5 % CO2 gezüchtet.

Nach Kultivierung über Nacht erfolgte ein Austausch durch frisches Gewebekulturmedium. Explantate wurden entweder in Anwesenheit oder Abwesenheit von Testproben kultiviert. Täglich wurde das Medium über einen Zeitraum von 10 Tagen gegen frisch ergänzte Testproben oder gegen Normalmedien ohne Testproben ausgetauscht.

Nach 10 Tagen Behandlung wurde ein Teil der Gewebeproben für die Extraktion von ECM-Proteinen aufgearbeitet.

Isolierte ECM wurden einer Proteinschätzung nach der BCA-Methode (Bicinchonic Acid) (Sigma Aldrich) unterzogen, und ELISAs (Biotechne) entsprechend dem Antigen Kollagen1A2 wurden gemäß dem Protokoll des Herstellers unter Verwendung gleicher Mengen an Proteinen durchgeführt, die aus dem BCA-Assay geschätzt wurden. Das Ablesen wurde in einem empfindlichen Mikroplatten-Lesegerät auf Filterbasis (Accuris, USA) durchgeführt. Die ELISA-Ergebnisse (% Unterschied) wurden in Prozent im Vergleich zur unbehandelten Kontrolle mit Mittelwert +/- SEM mit Wiederholungen in dreifacher Ausführung/Bedingung dargestellt.

Es wurden die folgenden Testproben wurden untersucht:
A. Glykolischer Kaviarextrakt enthaltend ca. 6% Fischeimaterial, 10% Wasser und ad 100 Propylenglykol. Dieser Extrakt wurde aus einer Extraktion mit Propylenglykol von einem Homogenisat aus Fischeiern von Acipenser baerii erhalten. Kommerziell ist dieser Extrakt unter dem Handelsnamen Caviar B Glycolysat CH von der Fa mibelle biochemistry group erhältlich.
B. Wässriger Kaviarextrakt umfassen ca. 21% Fischeimaterial, 1% Phenoxethanol, 5% Glycerin, 1% Glycin, 0,5% Xanthan Gum, 0,2% Galactaric Acid und ad 100 Wasser. Dieser Extrakt wurde aus einer Extraktion mit Wasser, Glycin, Xanthan Gum und Phenoxyethanol und Galactaric Acid von einem Homogenisat aus Fischeiern von Acipenser baerii erhalten. Kommerziell ist dieser Extrakt unter dem Handelsnamen Caviar B HydroEssence CH von der Fa mibelle biochemistry group erhältlich.
C. Lipophiler Kaviarextrakt enthaltend 40% Fischeimaterial, 0,02% Tocopherol, 1 Gew.-% Phenoxyethanol und ad 100 Triglyceride. Dieser Extrakt wurde aus einer Extraktion mit Triglyceriden und Phenoxyethanol von einem Homogenisat aus Fischeiern von Acipenser baerii erhalten. Kommerziell ist dieser Extrakt unter dem Handelsnamen Caviar B LipoEssence CH von der Fa mibelle biochemistry group erhältlich.
D. Mischung aus Glycerin, Water, Sodium Mannose-6 Phosphate und Mannose. Handelsprodukt Agefinity von der Fa. Givaudan. Der Anteil von Sodium Mannose-6 Phosphate beträgt 2,5 Gew.-%. Der Anteil von Mannose beträgt 2,5 Gew.-%.

In der nachfolgenden Tabelle sind die Einsatzkonzentrationen der Extrakte und der Mischung D angeben, welche genutzt wurden in dem Vergleichsversuch gegenüber einer unbehandelten Kontrolle.

| Testprobe | Expression von Kollagen I A2 in % relativ zur Kontrolle (100%) |
|---|---|
| Kontrolle (Ohne Zusatz) | 100% |
| 0,5% A | 73% |
| 0,5% B | 36% |
| 0,05% C | 98% |
| 1% D | 132% |
| 1% Mischung aus A, B, C und D, (Gewichtsverhältnisse A/B/C/D: 1/0,1/1/4) | 347% |

Die durchgeführten Messungen zeigen eindeutig, dass es einen Synergismus zwischen den Kaviarextrakten und dem Mannosephosphat eintritt. Die Zusätze Propylenglyckol, Glycerin, Glycin, Wasser, Phenoxyethanol, Wasser, Triglyceride und Galactaric Acid zeigen allein keine Steigerung der Expression von Kollagen I.

Im selben Screening wurde ebenfalls die Expression von Thrombospondin 1 durch Anwendung untersucht. Gegenüber einer Kontrolle zeigt die Proben mit dem glykolischen Extrakt A keine Steigerung der Expression von Thrombospondin 1 (Kontrolle 100%, Exrakt A 46%). Gleiches gilt für die Expression von Thrombospondin 1 bei Anwendung des wässrigen Extrakts B (65%). Bei Anwendung des lipophilen Extrakts C konnte eine Expression von Thrombospondin 1 von 299% in vergleich zur Kontrolle = 100% erzielt werde. Die Mischung D zeigte eine Expression von Thrombospondin 1 von 118%. Bei Anwendung der Mischung aus A, B, C und D, (Gewichtsverhältnisse A/B/C/D: 1/0,1/1/4) konnte eine überraschend starke Expression von 360% erzielt werden.

Alle gezeigten Messergebnisse für die Mischung A/B/C/D sind signifikant (p<0.05) verschieden gegenüber unbehandelt.

Nachfolgend sind weitere kosmetische Beispielrezepturen aufgeführt.

Beispielrezepturen:

| **Beispiel Nummer** | **1** | **2** | **3** | **4** | **5** |
|---|---|---|---|---|---|
| PEG-100 Stearate | 2,0 | 0,9 | | | |
| PEG-20 Glyceryl Stearate | | 1,1 | | | |
| PEG-40 Stearate | | | | | 1,0 |
| Ceteareth-25 | | | 0,5 | | |
| Steareth-100 | | | 0,5 | 2,0 | |
| Ceteth-20 | | | 1,0 | | |
| Myristyl Myristate | 1,0 | | | | 1,0 |
| Glyceryl Stearate | | 1,1 | | | 2,0 |
| Stearyl Alcohol | 2,0 | 1,0 | | | |
| Cetearyl Alcohol | | | | 4,0 | 2,5 |
| Cetyl Alkohol | 1,0 | | 3,0 | | |
| Hydrogenated Coco Glycerides | 2,0 | | | | |
| Butyrospermum Parkii (Shea) Butter | | 2,0 | | | 2,0 |
| C12-15 Alkyl Benzoate | | 3,0 | 2,0 | | 3,5 |
| Butylenglycol Dicaprylate/Dicaprate | 1,0 | | | 1,5 | |
| Caprylic/Capric Triglyceride | | 1,0 | 1,0 | 2,0 | 2,0 |
| Ethylhexyl Cocoate | 3,0 | | | | 1,5 |
| Octyldodecanol | | | 1,0 | | |
| Paraffinum Liquidum | | 1,0 | | | |
| Cera Microcristallina | 2,0 | | 1,0 | | 1,5 |
| Cyclomethicone | 4,1 | 1,0 | 4,0 | 3,5 | 5,0 |
| Dimethicone | | 2,3 | 1,0 | 1,2 | |
| Dicaprylyl Ether | 1,0 | 4,0 | 2,0 | | |
| Dicarprylyl Carbonate | | | | 2,8 | |
| Ethylhexyl Methoxycinnamate | 4,0 | 3,0 | 5,0 | 2,0 | 2,5 |
| Disodium Phenyl Dibenzimidazole Tetrasulfonate | 1,0 | 1,0 | 1,5 | 0,5 | 2,0 |
| Phenylbenzimidazole Sulphonic Acid | 2,0 | 3,0 | 1,0 | 1,5 | 1,5 |
| Ethylhexyl Triazone | | | | | 2,0 |
| Octocrylen | | | | 2,5 | |
| Ethylhexyl Salicylate | | | 1,0 | | |
| Wirkkomplex A+B+C+D (Gewichtsverhältnisse A/B/C/D: 1/0,1/1/4) | 0,1 | 0,2 | | | 0,5 |
| Wirkkomplex A+B+C+D (Gewichtsverhältnisse A/B/C/D: 0,8/0,5/1,2/3,4) | | | 0,1 | | |
| Wirkkomplex A+B+C+D (Gewichtsverhältnisse A/B/C/D: 1,2/0,3/0,9/4,1) | | | | 1 | |
| Biotin | | | | | 0,04 |
| Retinyl Palmitate | | | | 0,1 | |
| Thioctic Acid | 0,1 | | | | |
| Tocopheryl Acetate | | | 1,0 | | |
| Sodium Citrate | | 0.1 | | | |
| Sodium Ascorbyl Phosphate | 0,1 | | | | 0,1 |
| Trisodium EDTA | | 0.1 | | | |
| Phenoxyethanol | 0,4 | | 0,4 | 0,4 | 0,4 |
| Butylparaben | 0,6 | 0,3 | 0,2 | 0,3 | 0,3 |
| Alcohol Denat. | | 2,0 | | | |
| Xanthan Gum | 0,1 | | | | |
| Carbomer | 0,05 | | 0,1 | | 0,1 |
| Polyacrylamide | | 0,2 | | | |
| Glycerin | 10 | 6,0 | 6,5 | 7,5 | 8,0 |
| Butylene Glycol | 2,0 | 1,0 | | | |
| Füllstoffe/ Additive (Distärke-phosphat, SiO₂, BHT, Talkum, Aluminiumstearat) | 0,1 | 1,0 | 0,2 | 0,5 | 0,05 |
| Parfume | q.s. | q.s. | q.s. | q.s. | q.s. |
| Aqua | Ad 100 | Ad 100 | Ad 100 | Ad 100 | Ad 100 |

| **Beispiel Nummer** | **6** | **7** | **8** | **9** | **10** |
|---|---|---|---|---|---|
| PEG-50 Stearate | 2,5 | | | | 1,0 |
| PEG-40 Stearate | 1,0 | 1,0 | | | 0,5 |
| PEG-8 Stearate | | | | 1,0 | |
| PEG-8 Distearate | | | | | 1,0 |
| Glyceryl Stearate | | 3,0 | | | |
| Sorbitan Stearate | | 1,0 | | | |
| Steareth-21 | | | 2,0 | 1,0 | |
| Steareth-2 | | | 1,0 | | |
| Cetearyl Glucoside | | | | 2,0 | |
| Myristyl Myristate | | | | 1,0 | |
| Behenyl Alcohol | | 1,0 | | | 2,0 |
| Stearyl Alcohol | | | | 5,0 | |
| Cetearyl Alcohol | 3,0 | | 2,0 | | 1,0 |
| Cetyl Alcohol | | 1,0 | | | |
| Hydrogenated Coco Glycerides | 1,0 | | | | 1 |
| Butyrospermum Parkii (Shea) Butter | 2,5 | | | | |
| C12-15 Alkylbenzoate | | 2,0 | 5,0 | 2,5 | |
| Butylenglycol Dicaprylate/Dicaprate | 1,5 | | | | 2,0 |
| Caprylic/Capric Triglyceride | 1,0 | 1,5 | | 3,5 | |
| Ethylhexyl Cocoate | | | | | 2,0 |
| Octyldodecanol | | | 1,0 | | 1,5 |
| Paraffinum Liquidum | | | 1,0 | | |
| Cera Microcristallina | 1,8 | | | | |
| Cyclomethicone | 4,0 | 3,5 | 2,0 | 5,0 | 2,0 |
| Dimethicone | | | 2,0 | | 1,5 |
| Dicaprylyl Ether | | | 2,0 | | |
| Dicarprylyl Carbonate | | 2,0 | | 3,0 | 3,5 |
| N-(4-(2,4-Dihydroxyphenyl)thiazol-2-yl)-isobutyramid | 0,1 | 0,15 | 0,25 | 0,1 | 0,5 |
| Polydecene | | | | 4 | |
| Ethylhexyl Methoxycinnamate | 2,0 | 3,0 | 4,5 | 5,0 | 4,2 |
| Phenylbenzimidazole Sulphonic Acid | 0,5 | 2,0 | 2,0 | 3,3 | 1,0 |
| Disodium Phenyl Dibenzimidazole Tetrasulfonate | 1,0 | 1,0 | 1,5 | 2,3 | 0,5 |
| Wirkkomplex A+B+C+D (Gewichtsverhältnisse A/B/C/D: 1/0,1/1/4) | 0,2 | | 0,35 | | |
| Wirkkomplex A+B+C+D (Gewichtsverhältnisse A/B/C/D: 0,8/0,5/1,2/3,4) | | 1,1 | | | |
| Wirkkomplex A+B+C+D (Gewichtsverhältnisse A/B/C/D: 1,2/0,3/0,9/4,1) | | | | 0,8 | 0,7 |
| Biotin | | 0,02 | | | |
| Retinyl Palmitate | | | | 0,2 | |
| Tocopheryl Acetate | | 1,0 | | | 0,5 |
| Ascorbic Acid | | | 0,05 | | |
| Trisodium EDTA | | | 0,2 | 0,1 | |
| Phenoxyethanol | 0,5 | 0,4 | 0,5 | | 0,3 |
| Butylparaben | 0,1 | | | 0,4 | 0,6 |
| Ethylhexylglycerin | 0,2 | 0,2 | 0,1 | 0,4 | |
| Alcohol denat. | | 8,0 | | | 3,0 |
| Xanthan Gum | | 0,1 | | | |
| Gellan Gum | 0,2 | | 0,1 | | 0,1 |
| Carbomer | | 0,2 | | | |
| Glycerin | 10 | 5,0 | 6,0 | 4,0 | 7,0 |
| Butylene Glycol | | | | 2,0 | |
| Additive (Distärkephosphat, SiO₂, Talkum, BHT Aluminiumstearat) | 0,03 | | 0,05 | 3,0 | |
| Parfume | q.s. | q.s. | q.s. | q.s. | q.s. |
| Aqua | Ad 100 | Ad 100 | Ad 100 | Ad 100 | Ad 100 |

| | | | | | |
|---|---|---|---|---|---|
| **Beispiel Nummer** | **11** | **12** | **13** | **14** | **15** |
| PEG-100 Stearate | 1,4 | 0,1 | | 1,2 | |
| Glyceryl Stearate | 1,4 | 0,5 | 0,2 | 1,0 | 0,9 |
| Ceteareth-100 | | 2 | 3,1 | | 2,1 |
| Sorbitan Stearate | 2,3 | | | 2,5 | |
| Polysorbate 60 | 0,1 | | 0,3 | 0,8 | 0,7 |
| Polysorbate 80 | 0,8 | 0,5 | | | 0,1 |
| Sorbitane Isostearate | 0,1 | 0,25 | | 0,05 | |
| Theobroma Grandiflorum Seed Butter | 3,0 | | | 1,2 | 2,7 |
| Butyrospermum Parkii (Shea) Butter | | 2,5 | 3,5 | 1,9 | |
| Jojoba Esters | 2,0 | 2,5 | 1,3 | 0,5 | 1,0 |
| Beewax | 1,0 | 0,2 | 0,8 | 1,6 | 2,0 |
| Helianthus Annuus Seed Oil | 0,5 | | | 0,1 | |
| Persea Gratissima Oil | | 0,7 | | 0,3 | 0,5 |
| Olea Europaea Fruit Oil | | 0,1 | 0,6 | 0,1 | |
| Cyclomethicone | | 3,6 | 0,1 | 0,1 | 1,2 |
| Dimethicone | 5,4 | | 4,5 | 5,0 | 3,6 |
| Squalane | 3,0 | | | | 1,9 |
| Carbomer | | 0,2 | 0,4 | | 0,15 |
| Hydroxyethyl Acrylate/Sodium Acryloydimethyl Taurate Copolymer | 1,7 | 0,1 | 1,0 | 2,0 | 0,1 |
| Polymethyl Methacrylate | 0,5 | 0,6 | 0,1 | | |
| Dimethicone Crosspolymer | 0,6 | 0,3 | | 0,25 | |
| Pullulan | 0,5 | | 0,4 | | |
| Carrageenan | 0,2 | 0,3 | | 0,7 | 0,3 |
| Caprylyl Glycol | 0,3 | | 0,1 | 0,25 | |
| Methylpropanediol | | 1,3 | | | |
| Glycerin | 3,1 | 1,7 | 4,0 | 2,8 | 3,5 |
| Sorbitol | | | | 0,1 | |
| Propanediol | 2,0 | | 1,9 | | |
| Propylene Glycol | 0,1 | | 0,1 | 0,2 | 0,3 |
| Pentylene Glycol | 0,1 | 0,5 | | | |
| Butylene Glycol | 0,2 | | 0,3 | 0,2 | |
| Tocopherol Acetate | 0,5 | 0,2 | | 0,45 | 0,35 |
| Ubichinone | | 0,1 | 0,2 | | 0,15 |
| Retinyl Palmitate | | 0,15 | | 0,15 | |
| Wirkkomplex A+B+C+D (Gewichtsverhältnisse A/B/C/D: 1/0,1/1/4) | | | 0,65 | | 0,01 |
| Wirkkomplex A+B+C+D (Gewichtsverhältnisse A/B/C/D: 0,8/0,5/1,2/3,4) | | 0,7 | | | |
| Wirkkomplex A+B+C+D (Gewichtsverhältnisse A/B/C/D: 1,2/0,3/0,9/4,1) | 0,25 | | | 0,35 | |
| Disodium EDTA | 0,1 | 0,1 | 0,1 | 0,1 | 0,1 |
| Triethanolamine | 0,1 | 0,1 | 0,1 | 0,1 | 0,1 |
| Phenoxyethanol | 0,4 | 0,3 | 0,5 | 0,3 | 0,45 |
| Ethylhexylglycerin | | 0,2 | | 0,1 | 0,05 |
| Titanium Dioxide | 0,3 | 0,1 | | | 0,2 |
| Tapioca Starch | | | 0,05 | | |
| Talkum | | 0,05 | | | |
| Parfume | q.s. | q.s. | q.s. | q.s. | q.s. |
| Aqua | Ad 100 | Ad 100 | Ad 100 | Ad 100 | Ad 100 |

## Patentansprüche

1. Wirkstoffkombination enthaltend
a) mindestens einen Kaviarextrakt, und
b) Mannosephosphat und/oder Natrium Mannosephosphat.

2. Wirkstoffkombination nach Anspruch 1 **dadurch gekennzeichnet, dass** der Kaviarextrakt aus Fischeiern des Weißen Störs (Acipenser transmontanus) und/oder des Sibirische Störs (Acipenser baerii) erhalten wird.

3. Wirkstoffkombination nach einem der vorhergehenden Ansprüche **dadurch gekennzeichnet, dass** der oder die Kaviarextrakte erhalten werden durch ein Verfahren umfassend die Schritte
1) Homogenisierung der bereitgestellten Fischeier in mindestens einem Lösungsmittel,
2) Extraktion mindestens einer flüssigen Phase aus dem unter 1) erhaltenen Homogenisat zum Erhalt des Extrakts, und
3) Optional einer Filtration des unter 2) gewonnenen Extrakts, um feste Schwebstoffe aus
4) dem Extrakt zu entfernen.

4. Wirkstoffkombination nach Anspruch 3 **dadurch gekennzeichnet, dass** ein Kaviarextrakt in der Wirkstoffkombination enthalten ist, bei dessen Herstellung mindestens Öl als Lösemittel bei der Homogenisation der Fischeier zugesetzt werden und bei dem die Ölphase extrahiert wird.

5. Wirkstoffkombination nach Anspruch 4 **dadurch gekennzeichnet, dass** als Öl Triglyceride, insbesondere Caprylic/Capric Triglyceride, eingesetzt werden.

6. Wirkstoffkombination nach einem der Anspruch 3 bis 5 **dadurch gekennzeichnet, dass** mindestens ein Kaviarextrakt enthalten ist, bei dessen Herstellung mindestens Wasser und/oder ein Glykol als Lösemittel bei der Homogenisation der Fischeier zugesetzt werden.

7. Wirkstoffkombination nach Anspruch 6 **dadurch gekennzeichnet, dass** die wässrige Phase zum Erhalt des eines Kaviarextrakts extrahiert wird.

8. Wirkstoffkombination nach einem der vorhergehenden Ansprüche, **dadurch gekennzeichnet, dass** mindestens zwei verschiedene Kaviarextrakte enthalten sind.

9. Wirkstoffkombination nach Anspruch 8 **dadurch gekennzeichnet, dass** ein erster lipophiler Kaviarextrakt eingesetzt wird, bei dessen Herstellung mindestens Öl, vorteilhaft Caprylic/Capric Triglyceride, als Lösemittel bei der Homogenisation der Fischeier zugesetzt werden und bei dem die Ölphase zum Erhalt des Extrakt extrahiert wird, und wobei ein zweiter hydrophiler oder glykolischer Kaviarextrakt eingesetzt wird, bei dessen Herstellung mindestens Wasser und/oder ein Glykol als Lösemittel bei der Homogenisation der Fischeier zugesetzt werden und bei dem die wässrige Phase zum Erhalt des zweiten Extrakts extrahiert wird.

10. Wirkstoffkombination nach einem der Ansprüche 3 bis 9 **dadurch gekennzeichnet, dass** drei verschiedene Kaviarextrakte eingesetzt werden, wobei
- ein erster lipophiler Kaviarextrakt eingesetzt wird, bei dessen Herstellung mindestens Öl, vorteilhaft Caprylic/Capric Triglyceride, als Lösemittel bei der Homogenisation der Fischeier zugesetzt werden und bei dem die Ölphase zum Erhalt des lipophilen Kaviarextrakts extrahiert wird,
- ein zweiter wässriger Kaviarextrakt eingesetzt wird, bei dessen Herstellung mindestens Wasser, jedoch kein Glykol, als Lösemittel bei der Homogenisation der Fischeier zugesetzt werden und bei dem die wässrige Phase zum Erhalt des wässrigen Kaviarextrakts extrahiert wird, und
- ein dritter glykolischer Kaviarextrakt eingesetzt wird, bei dessen Herstellung mindestens Glykol als Lösemittel bei der Homogenisation der Fischeier zugesetzt werden und bei dem die glykolische Phase zum Erhalt des dritten Kaviarextrakts extrahiert wird.

11. Wirkstoffkombination nach einem der Ansprüche 4 bis 10 **dadurch gekennzeichnet, dass** der Gewichtsanteil der Fischeier zur zugegebenen Ölphase bei der Homogenisation von 1:0,1 bis 1:1 und insbesondere von 1:0,2 bis 1:0,5 beträgt.

12. Wirkstoffkombination nach einem der Ansprüche 3 bis 11 **dadurch gekennzeichnet, dass** Wasser für die Homogenisation zugesetzt und der Gewichtsanteil der Fischeier zu der durch Zugabe von Wasser entstandenen wässrigen Phase von 1:10 bis 1:1, bevorzugt von 1:8 bis 1:2 und insbesondere bevorzugt von 1:5 bis 1:3 beträgt.

13. Wirkstoffkombination nach einem der Ansprüche 9 bis 12 **dadurch gekennzeichnet, dass** der Gewichtsanteil der Fischeier zum zugegebenen Glycol, insbesondere zu Propylenglycol, von 1:0,1 bis 1:100 und insbesondere von 1:0,2 bis 1:20 und insbesondere 1:0,5 bis 1:10 beträgt.

14. Wirkstoffkombination nach einem der Ansprüche 9 bis 13 **dadurch gekennzeichnet, dass** Gewichtsverhältnis vom lipophilen Kaviarextrakt zum wässriger Kaviarextrakt von 50:1 bis 1:10, bevorzugt 30:1 bis 1:1 und insbesondere bevorzugt 15:1 bis 5:1 beträgt.

15. Wirkstoffkombination nach einem der Ansprüche 10 bis 13 **dadurch gekennzeichnet, dass** das Gewichtsverhältnis vom lipophilen Kaviarextrakt zum glykolischen Kaviarextrakt von 50:1 bis 1:50, bevorzugt 20:1 bis 1:20 und insbesondere bevorzugt 10:1 bis 1:10 beträgt.

16. Wirkstoffkombination nach einem der vorhergehenden Ansprüche **dadurch gekennzeichnet, dass** das Gewichtsverhältnis von der Gesamtheit der Kaviarextrakte zu Mannosephosphat und/oder Natrium Mannosephosphat von 200:1 bis 1:50, bevorzugt 100:1 bis 1:10, bevorzugt von 50:1 bis 1:1 und insbesondere bevorzugt von 30:1 bis 2:1 beträgt.

17. Wirkstoffkombination nach einem der vorhergehenden Ansprüche **dadurch gekennzeichnet, dass** als Natrium Mannosephosphat das Natrium Mannose-6-Phosphat enthalten ist und/oder als Mannosephosphat Mannose-6-phosphat enthalten ist.

18. Wirkstoffkombination nach einem der vorhergehenden Ansprüche **dadurch gekennzeichnet, dass** die Wirkstoffkombination Mannose umfasst, wobei vorteilhaft das Gewichtsverhältnis von der Gesamtheit der erfindungsgemäßen Kaviarextrakte zu Mannose von 200:1 bis 1:50, bevorzugt 100:1 bis 1:10, bevorzugt von 50:1 bis 1:1 und insbesondere bevorzugt von 30:1 bis 2:1.

19. Kosmetische Zubereitung enthaltend eine Wirkstoffkombination nach einem der Ansprüche 1 bis 18.

20. Verwendung der Wirkstoffkombination gemäß einem der Ansprüche 1 bis 18 in kosmetischen Zubereitungen.

21. Verwendung der Wirkstoffkombination gemäß einem der Ansprüche 1 bis 18 zur Steigerung der Expression von Kollagen I sowie zur Reduktion von Hautalterserscheinungen, insbesondere zur Reduktion von Hautalterserscheinungen, wie Schlaffheit, Falten und Festigkeits- und Volumenverlust.

22. Verwendung der Wirkstoffkombination gemäß einem der Ansprüche 1 bis 18 zur Stärkung der Retinaculum cutis und/oder der Bindegewebsstränge der menschlichen Haut.
